# EUROPEAN PATENT APPLICATION

(11) **EP 3 696 042 A1**
(43) Date of publication of application: **19.08.2020**
(21) Application number: 18867118.4
(22) Date of filing: 01.10.2018
(51) Int. Cl.: B60W 50/12, A61B 5/18, B62J 27/00, B62J 99/00

(54) **VEHICLE, DETERMINATION METHOD, AND DETERMINATION PROGRAM**

(30) Priority: 12.10.2017 JP 2017198639
(71) Applicant: Kyocera Corporation, Kyoto-shi, Kyoto 612-8501 (JP)
(72) Inventor: TONG, Fangwei, Kyoto-shi Kyoto 612-8501 (JP); HIGUCHI, Takeshi, Kyoto-shi Kyoto 612-8501 (JP)
(74) Representative: Viering, Jentschura & Partner mbB Patent- und Rechtsanwälte
(86) International application number: PCT/JP2018/036721
(87) International publication number: WO 2019/073845

(57) **Abstract**

A vehicle includes an information acquisition interface that acquires operation information resulting from a control action performed by a driver and a controller that judges a degree of fatigue of the driver based on the operation information.

## Description

### CROSS-REFERENCE TO RELATED APPLICATION

The present application claims priority to and the benefit of Japanese Patent Application No. 2017-198639 filed October 12, 2017, the entire contents of which are incorporated herein by reference.

### TECHNICAL FIELD

The present disclosure relates to a vehicle, a judgment method, and a judgment program.

### BACKGROUND

An apparatus used for driving safety of a vehicle is known. For example, an apparatus for acquiring data for a safety apparatus of a balance vehicle is disclosed in patent literature (PTL) 1. A driver danger management apparatus that predicts danger based on biological information of the driver and moveable body information is disclosed in PTL 2.

### CITATION LIST

### Patent Literature

PTL 1: JP2013-186897A
PTL 2: JP2016-018314A

### SUMMARY

A vehicle according to an aspect includes an information acquisition interface configured to acquire operation information resulting from a control action performed by a driver and a controller configured to judge a degree of fatigue of the driver based on the operation information.

A judgment method according to an aspect includes acquiring, by a controller using an information acquisition interface, operation information resulting from a control action performed by a driver and judging, by the controller, a degree of fatigue of the driver based on the operation information.

A judgment program according to an aspect is for causing a computer to acquire operation information resulting from a control action performed by a driver and judge a degree of fatigue of the driver based on the acquired operation information.

### BRIEF DESCRIPTION OF THE DRAWINGS

In the accompanying drawings:
FIG. 1 is a functional block diagram illustrating an example schematic configuration of an information processing system according to an embodiment;
FIG. 2 is a schematic side view illustrating an example of the vehicle of FIG. 1;
FIG. 3A illustrates an example of a brake operation performed by a driver;
FIG. 3B illustrates an example of the change in speed of a vehicle due to the brake operation of FIG. 3A;
FIG. 3C illustrates an example of the change in acceleration of a vehicle due to the brake operation of FIG. 3A;
FIG. 4A illustrates an example of a brake operation performed by a driver;
FIG. 4B illustrates an example of the change in speed of a vehicle due to the brake operation of FIG. 4A;
FIG. 4C illustrates an example of the change in acceleration of a vehicle due to the brake operation of FIG. 4A;
FIG. 5 is a flowchart illustrating an example of a process executed by the controller of the vehicle in FIG. 1;
FIG. 6 is a flowchart illustrating an example of a process executed by the controller of the vehicle in FIG. 1; and
FIG. 7 is a flowchart illustrating a modification of a process executed by the controller of the vehicle in FIG. 1.

### DETAILED DESCRIPTION

Embodiments are described below in detail with reference to the drawings.

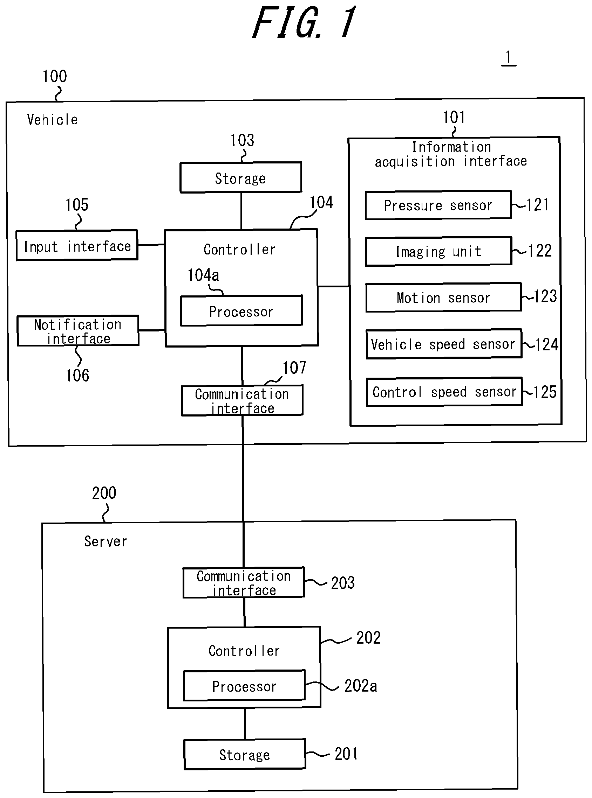

FIG. 1 is a functional block diagram illustrating an example configuration of an information processing system 1 according to an embodiment. As illustrated in FIG. 1, the information processing system 1 includes a vehicle 100 and a server 200. The vehicle 100 and the server 200 are communicably connected to each other.

The vehicle 100 may, for example, be a car such as an electric car, a hybrid electric car, and a gasoline car; a two-wheeled vehicle such as a motorcycle; a bicycle; or the like. In the present embodiment, the vehicle 100 is described below as being a two-wheeled vehicle.

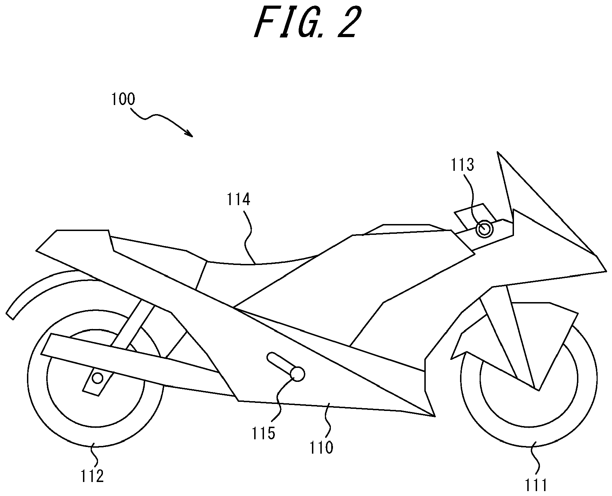

FIG. 2 is a schematic side view illustrating an example of the vehicle 100 according to the present embodiment. The vehicle 100 includes a body 110, a front wheel 111 and rear wheel 112 supporting the body 110, and a handlebar 113 for steering.

A fuel tank storing fuel, an engine to drive the vehicle 100, and the like are disposed in the body 110. The body 110 includes a seat 114 where the driver sits when driving. In other words, the driver sits in the seat 114 and drives the vehicle 100. Furthermore, the body 110 includes a brake pedal for performing an operation to apply the brake and a shift pedal for performing an operation to change gears. In the present embodiment, the brake pedal and the shift pedal are indicated together as a pedal 115. The body 110 may include a mechanism in which the functional blocks of FIG. 1 are implemented.

The handlebar 113 includes a brake lever for performing an operation to apply the brake and a clutch lever for performing a clutch operation. The handlebar 113 may, for example, be provided at the front side of the body 110.

Referring again to FIG. 1, the vehicle 100 includes an information acquisition interface 101, a storage 103, a controller 104, an input interface 105, a notification interface 106, and a communication interface 107 as functional blocks.

The information acquisition interface 101 acquires information resulting from a control action performed by the driver (operation information). The control action may, for example, encompass a steering operation, an accelerator operation, a brake operation, and a gear operation performed by the driver.

The information acquisition interface 101 is configured to include various sensors for acquiring operation information. In the example in FIG. 1, for example, the information acquisition interface 101 is configured to include a pressure sensor 121, an imaging unit 122, a motion sensor 123, a vehicle speed sensor 124, and a control speed sensor 125 as sensors for acquiring operation information.

The pressure sensor 121 measures the pressure at a predetermined position of the vehicle 100 due to driver operation. The pressure sensor 121 measures the strength (pressure) with which the driver grips the handlebar, for example. The pressure sensor 121 measures the strength (pressure) with which the driver steps on the pedal 115, for example. The pressure sensor 121 is not limited to these examples and may be disposed to allow measurement of pressure on any position of the vehicle 100 due to driver operation. A signal of information related to the pressure measured by the pressure sensor 121 is transmitted to the controller 104. The information related to pressure measured by the pressure sensor 121 may be stored in the storage 103.

The imaging unit 122 captures an image of the driver seated in the seat 114 and driving the vehicle 100. The imaging unit 122 may, for example, be configured by a digital video camera. A signal of the image captured by the imaging unit 122 is transmitted to the controller 104. The image captured by the imaging unit 122 may be stored in the storage 103.

The motion sensor 123 detects motion of the vehicle 100. The motion sensor 123 is configured by an acceleration sensor, for example, and detects the direction, magnitude, and the like of acceleration acting on the vehicle 100 as the motion of the vehicle 100. The signal of information related to motion detected by the motion sensor 123 is transmitted to the controller 104. The information related to the motion detected by the motion sensor 123 may be stored in the storage 103.

The motion sensor 123 is not, however, limited to being an acceleration sensor and may be configured as any sensor capable of detecting motion of the vehicle 100. For example, the motion sensor 123 may be configured by an angular velocity sensor, an angle sensor, or the like. The motion sensor 123 may be configured by a plurality of types of sensors.

The motion sensor 123 may, for example, be disposed in the handlebar 113 and detect motion of the handlebar 113.

The vehicle speed sensor 124 detects the driving speed of the vehicle 100. The vehicle speed sensor 124 may be a type of sensor that directly detects the speed or may be a type of sensor that calculates the speed based on the engine rotation speed and the gear ratio. The signal of information related to speed detected by the vehicle speed sensor 124 is transmitted to the controller 104. The information related to the motion detected by the vehicle speed sensor 124 may be stored in the storage 103.

The control speed sensor 125 detects the speed at the time a predetermined operation position in the vehicle 100 is operated. The operation position is a position operated by the hand, foot, or the like of the driver and may, for example, include the handlebar 113 and the pedal 115. For example, when the operation position is the pedal 115, the control speed sensor 125 detects the speed at which the pedal 115 is operated, i.e. the speed of displacement due to the pedal 115 being pressed. The control speed sensor 125 may be disposed to allow detection of speed at a predetermined operation position.

The information acquisition interface 101 need not include all of the sensors indicated in the present disclosure and the example of FIG. 1. The information acquisition interface 101 may also be configured to include sensors other than those indicated in the present disclosure and the example of FIG. 1.

It suffices for the various sensors in the information acquisition interface 101 to be disposed at positions allowing acquisition of information detected by each sensor. The pressure sensor 121 that measures the strength (pressure) with which the driver grips the handlebar may be disposed in the grip of the handlebar 113, for example. The pressure sensor 121 that measures the strength (pressure) with which the driver steps on the pedal 115 may be disposed in the pedal 115, for example. The same is true for other sensors as well.

The storage 103 can be configured by a semiconductor memory, a magnetic memory, or the like. The storage 103 stores various information, programs for operating the vehicle 100, and the like. The storage 103 may also function as a working memory. The storage 103 may store the various information acquired by the information acquisition interface 101 in association with the time of acquisition. The storage 103 may store the information acquired by the sensors of the information acquisition interface 101 in association with the time of acquisition.

The storage 103 may store a pattern of the operation information of the driver. Details on the pattern of the operation information of the driver are provided below. The storage 103 may store operation information of the driver when the driver starts driving the vehicle 100. The start of driving referred to here includes a predetermined time after the driver starts driving the vehicle 100 (such as five minutes after the driver starts driving). The storage 103 may store the result of the judgment, described below, made by the controller 104.

The controller 104 includes at least one processor 104a that controls and manages the vehicle 100 overall, starting with the functional blocks of the vehicle 100. The controller 104 is configured to include and implement the functions of at least one processor 104a, such as a central processing unit (CPU), that executes programs prescribing control procedures. Such programs may, for example, be stored in the storage 103 or on an external storage medium or the like connected to the vehicle 100.

In various embodiments, the one or more processors 104a may be implemented as a single integrated circuit (IC) or as a plurality of communicatively connected integrated circuits and/or discrete circuits. The at least one processor 1104a can be implemented with a variety of known techniques.

In an embodiment, the processor 104a includes one or more circuits or units configured to execute one or more data calculation procedures or processes by executing instructions stored in related memory, for example. In another embodiment, the processor 104a may be firmware (such as discrete logic components) configured to execute one or more data calculation procedures or processes.

In various embodiments, the processor 104a may include one or more processors, controllers, microprocessors, microcontrollers, application specific integrated circuits (ASIC), digital signal processors, programmable logic devices, field programmable gate arrays, any combination of these devices or structures, or a combination of other known devices or structures to implement the functions of the controller 104, described below.

The controller 104 judges the degree of fatigue of the driver based on the operation information of the driver acquired by the information acquisition interface 101. The degree of fatigue is the extent of fatigue. The degree of fatigue may, for example, be represented as a numerical value. The degree of fatigue may, for example, be represented by stages. The degree of fatigue may, for example, be represented by two stages, "high" and "low". The controller 104 may, for example, judge the degree of fatigue resulting from the driver driving the vehicle 100.

The controller 104 judges the degree of fatigue by, for example, judging whether the operation information of the driver exhibits the normal pattern of the driver. The controller 104 judges the degree of fatigue by referring to the pattern of the operation information of the driver stored in the storage 103 and judging whether the operation information of the driver exhibits the normal pattern of the driver. Details of the judgment process executed by the controller 104 are provided below.

The input interface 105 receives operation input from the driver, for example. The input interface 105 is configured using operation buttons (operation keys), for example. The input interface 105 may be formed by a touchscreen and receive touch operation input from the user to an input region, displayed on a portion of a display device, for receiving operation input. The input interface 105 may be provided near the position where gauges are disposed at the front of the body 110, for example.

The notification interface 106 provides notification of information by sound, vibration, images, and the like. The notification interface 106 may be configured to include a speaker and an oscillator or the like. In response to control by the controller 104, the notification interface 106 provides notification of the result of the judgment process by the controller 104, for example. In other words, the notification interface 106 provides notification of the judgment result related to the degree of fatigue of the driver. The notification interface 106 may output a notification encouraging the driver to rest when the degree of fatigue of the driver exceeds a predetermined threshold, for example.

The communication interface 107 transmits and receives various information by communicating with the server 200. The communication interface 107 can transmit and receive information using a network that is wireless, wired, or a combination of wireless and wired. The communication interface 107 can, for example, communicate by Bluetooth® (Bluetooth is a registered trademark in Japan, other countries, or both), infrared, near field radio communication (NFC), a wireless local area network (LAN), a wired LAN, any other communication medium, or any combination thereof.

The server 200 is, for example, configured by a computer. The server 200 acquires information from the vehicle 100 and stores the acquired information. The server 200 may, for example, provide (transmit) the stored information to a non-illustrated terminal apparatus or the like. Terminal apparatuses may, for example, include mobile phones, smartphones, tablets, or the like. The terminal apparatus may, for example, be a terminal apparatus possessed by a related party that has a predetermined relationship with the driver. The related party may, for example, be a relative of the driver, the driver's primary doctor, or the like. When, for example, the vehicle 100 is used for competition such as a car race, the related party may be a team member, a cheerleader, or a coach of the competition team to which the driver belongs, for example.

The server 200 includes a storage 201, a controller 202, and a communication interface 203.

The storage 201 can be configured by a semiconductor memory, a magnetic memory, or the like. The storage 201 stores various information, programs for operating the server 200, and the like. The storage 201 may also function as a working memory. The storage 201 may store information acquired from the vehicle 100. For example, the storage 201 may store the judgment result related to the degree of fatigue of the driver.

The controller 202 includes at least one processor 202a that controls and manages the server 200 overall, starting with the functional blocks of the server 200. The functions of the controller 202 are implemented by the at least one processor 202a, which is a CPU or the like that executes programs prescribing control procedures. Such programs may, for example, be stored in the storage 201 or on an external storage medium or the like connected to the server 200. The examples listed in the description of the processor 104a may be used as the specific configuration of the processor 202a.

The communication interface 203 transmits and receives various information by communicating with the vehicle 100. The communication interface 203 can transmit and receive information using a network that is wireless, wired, or a combination of wireless and wired. The communication interface 203 can, for example, communicate with Bluetooth®, infrared, NFC, wireless LAN, wired LAN, any other communication medium, or any combination of these.

Next, the judgment process executed by the controller 104 is described in detail along with the pattern of operation information of the driver. For the sake of simplicity, the operation information is described here as being information related to a brake operation. The information related to a brake operation may include information related to the speed at which the driver operates (presses) the brake pedal.

When people are fatigued, their concentration and attentiveness are reduced, and they become weaker. For example, when a person is driving the vehicle 100 and grows fatigued, the person may notice obstacles such as people or objects later than when not fatigued, due to a reduction in concentration and attentiveness. If the vehicle 100 is to be stopped, for example, after an obstacle is discovered late, then the vehicle 100 needs to be stopped over a shorter distance, and the driver brakes hard. In other words, when a person is fatigued, the speed with which the brake pedal is pressed changes, and the frequency of hard braking increases, as compared to when the person is not fatigued. The controller 104 according to the present embodiment judges the degree of fatigue of the driver based on the speed with which the brake pedal is pressed, the frequency of hard braking, and the like, for example. In the example described in the present embodiment, the controller 104 judges the degree of fatigue of the driver based on the speed with which the brake pedal is pressed.

When the controller 104 judges the degree of fatigue of the driver based on the speed with which the brake pedal is pressed, the storage 103 stores a pattern of operation information related to a brake operation of the driver when the degree of fatigue of the driver is lower than a predetermined value, for example. This operation information related to a brake operation of the driver when the degree of fatigue of the driver is lower than a predetermined value is also referred to as "normal operation information" in the present disclosure. The controller 104 judges the degree of fatigue of the driver by judging whether the operation information of the driver while the driver is driving the vehicle 100 is included in the range of a pattern of normal operation information stored in the storage 103. The controller 104 can, for example, judge that the degree of fatigue of the driver is low when the operation information of the driver is judged to be included in the range of the pattern of normal operation information stored in the storage 103. The controller 104 can, for example, judge that the degree of fatigue of the driver is high when the operation information of the driver is judged not to be included in the range of the pattern of normal operation information stored in the storage 103.

During use of the information processing system 1, the controller 104 executes a storage process to store at least the pattern of normal operation information of the driver in the storage 103. This storage process can be expressed as a learning process in which the controller 104 learns. Accordingly, the storage process in the present disclosure can be replaced by a learning process, and simultaneously, the term "storage" can be replaced by the term "learning". The storage process may be executed by any suitable method. For example, when a driver gets in the vehicle 100 and takes a short test drive, such as several minutes, the controller 104 may store the normal pattern during the test drive in the storage 103. The test drive may, for example, be taken when the driver considers himself not to be fatigued. During the test drive, the information acquisition interface 101 acquires the pattern of normal operation information of the driver. For example, when the operation information is information related to a brake operation, the controller 104 acquires the speed with which the driver presses the brake pedal during the test drive using the control speed sensor 125. The controller 104 stores the operation information acquired by the control speed sensor 125 during the test drive in the storage 103 as normal operation information.

When at least the normal pattern is stored in the storage 103, the controller 104 can execute the judgment process to judge whether the operation information of the user is included in the range of the pattern of normal operation information. For example, the controller 104 acquires operation information of the brake petal detected by the control speed sensor 125 during driving of the vehicle 100. The controller 104 judges whether the acquired operation information is included in the range of the pattern of normal operation information stored in the storage 103. The range of the pattern of normal operation information is not limited to the pattern of normal operation information stored in the storage 103 and may include a pattern similar to the pattern of normal operation information. Inclusion in the range of the pattern of normal operation information may be judged based on whether the operation information of the driver is within the range of a predetermined threshold relative to the pattern of normal operation information stored in the storage 103. The controller 104 can judge that the degree of fatigue of the driver is high when the operation information of the driver detected by the control speed sensor 125 is judged not to be included in the range of the pattern of normal operation information stored in the storage 103. The controller 104 can judge that the degree of fatigue of the driver is low when the operation information of the driver detected by the control speed sensor 125 is judged to be included in the range of the pattern of normal operation information stored in the storage 103. The controller 104 may judge the degree of fatigue of the driver in accordance with the degree of difference between the operation information of the driver detected by the control speed sensor 125 and the pattern of normal operation information stored in the storage 103. For example, the controller 104 may judge that the degree of fatigue of the driver is higher as the operation information of the driver detected by the control speed sensor 125 is more distant from the pattern of normal operation information stored in the storage 103.

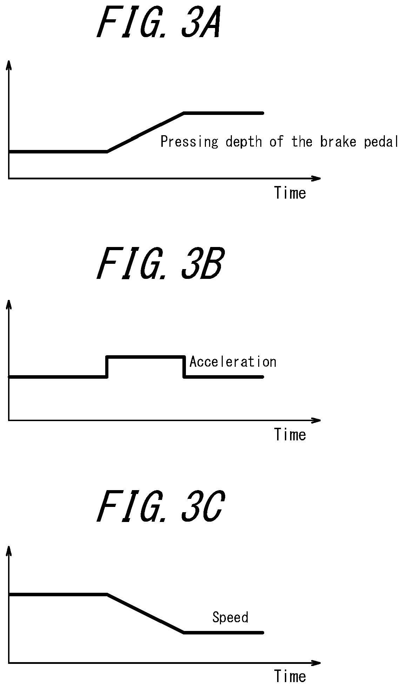

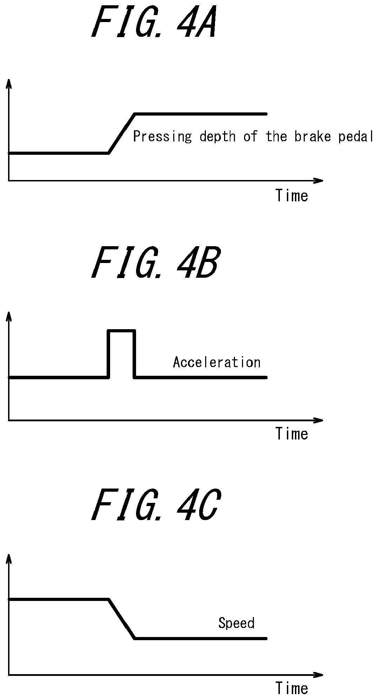

FIGS. 3A, 3B, 3C and FIGS. 4A, 4B, 4C illustrate examples of brake operations by the driver and changes in speed and acceleration of the vehicle 100. FIG. 3A illustrates the change in the pressing depth of the brake pedal when the degree of fatigue of the driver is judged to be low, for example. In FIG. 3A, the horizontal axis represents time, and the vertical axis represents the pressing depth of the brake pedal. FIG. 3B illustrates the change in acceleration of the vehicle 100 when the driver presses the brake pedal as illustrated in FIG. 3A. In FIG. 3B, the horizontal axis represents time, and the vertical axis represents acceleration. FIG. 3C illustrates the change in speed of the vehicle 100 when the driver presses the brake pedal as illustrated in FIG. 3A. In FIG. 3C, the horizontal axis represents time, and the vertical axis represents speed. FIG. 4A illustrates the change in the pressing depth of the brake pedal when the degree of fatigue of the driver is judged to be high, for example. In FIG. 4A, the horizontal axis represents time, and the vertical axis represents the pressing depth of the brake pedal. FIG. 4B illustrates the change in acceleration of the vehicle 100 when the driver presses the brake pedal as illustrated in FIG. 4A. In FIG. 4B, the horizontal axis represents time, and the vertical axis represents acceleration. FIG. 4C illustrates the change in speed of the vehicle 100 when the driver presses the brake pedal as illustrated in FIG. 4A. In FIG. 4C, the horizontal axis represents time, and the vertical axis represents speed. The changes in the pressing depth of the brake pedal in FIGS. 3A and 4A are examples of the above-described control information.

When the driver gently presses the brake pedal as illustrated in FIG. 3A, the vehicle 100 gently slows down, as illustrated in FIG. 3C. When the driver abruptly presses the brake pedal as illustrated in FIG. 4A, the vehicle 100 abruptly slows down, as illustrated in FIG. 4C. FIGS. 4A, 4B, 4C illustrate the state of hard braking. When, for example, the driver has a low degree of fatigue and high attentiveness, the driver has ample time to press the brake pedal gently, as illustrated in FIG. 3A. On the other hand, when the driver has a high degree of fatigue and reduced attentiveness, for example, the driver tends to brake hard, as illustrated in FIG. 4A.

The controller 104 refers to the pattern of normal operation information stored in the storage 103, for example, and when the operation information of the driver detected by the control speed sensor 125 is like the operation information illustrated in FIG. 3A, the controller 104 judges that the operation information is included in the range of the pattern of normal operation information. In this case, the controller 104 judges that the degree of fatigue of the driver is low.

The controller 104 refers to the pattern of normal operation information stored in the storage 103, for example, and when the operation information of the driver detected by the control speed sensor 125 is like the operation information illustrated in FIG. 4A, the controller 104 judges that the operation information is included_{[BW1]} in the range of the pattern of normal operation information. In this case, the controller 104 judges that the degree of fatigue of the driver is high.

The controller 104 may provide notification, from the notification interface 106, of the judgment result related to the degree of fatigue of the driver. The controller 104 may be configured only to provide notification, from the notification interface 106, of a high degree of fatigue of the driver when the degree of fatigue of the driver is judged not to be included in the pattern of normal operation information. The driver can learn what his own degree of fatigue is from the notification. When the driver is notified of a high degree of fatigue by the notification interface 106, the driver can take measures to ensure safety, such as resting, even if the driver is not conscious of being fatigued.

The controller 104 can transmit the judgment result related to the degree of fatigue of the driver to the server 200 via the communication interface 107. The controller 104 may be configured to transmit the judgment result to the server 200 only when the degree of fatigue of the driver is judged not to be included in the pattern of normal operation information. The server 200 stores the judgment result acquired from the vehicle 100 in the storage 201. The server 200 may store the acquired judgment result in the storage 201 in association with identification information, such as an ID that uniquely identifies the driver. The server 200 can store judgment results related to a plurality of drivers. The server 200 may transmit the judgment result to a terminal apparatus possessed by a party related to the driver.

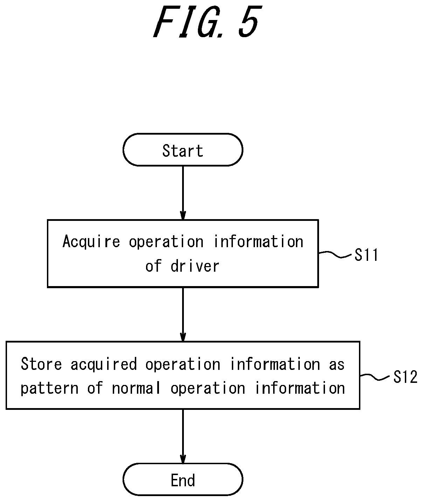

The control executed by the controller 104 is further described with reference to flowcharts. FIG. 5 is a flowchart illustrating an example of a process executed by the controller 104 of the vehicle 100. FIG. 5 illustrates an example of the storage process. The flowchart of FIG. 5 is, for example, executed during a test drive. In other words, the driver uses the input interface 105 to input the start of a test drive when the driver takes the vehicle 100 on a test drive. The controller 104 starts the flow of FIG. 5 in response to the input.

The controller 104 acquires operation information of the driver, detected by the sensors of the information acquisition interface 101 during the test drive, from the sensors (step S11). For example, the controller 104 acquires information related to the speed with which the brake pedal is pressed, detected by the control speed sensor 125, as the operation information.

The controller 104 stores the operation information acquired in step S11 as a pattern of normal operation information in the storage 103 (step S12). In this way, the pattern of normal operation information is stored (accumulated) in the storage 103 by a test drive.

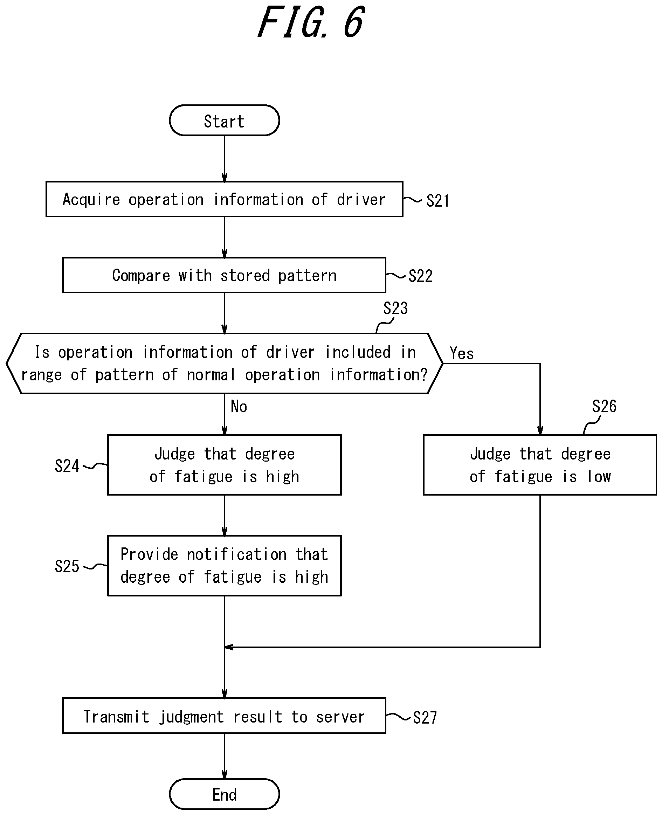

FIG. 6 is a flowchart illustrating an example of a process executed by the controller 104 of the vehicle 100. FIG. 6 illustrates an example of the judgment process. The flowchart in FIG. 6 is executed in the case of the pattern of normal operation information being stored in the storage 103 by the test drive, for example.

The controller 104 acquires operation information of the driver, detected by the sensors of the information acquisition interface 101, from the sensors while the driver is driving the vehicle 100 (step S21). For example, the controller 104 acquires information related to the speed with which the brake pedal is pressed, detected by the control speed sensor 125, as the operation information.

The controller 104 compares the operation information of the driver acquired in step S21 with the pattern stored in the storage 103 (step S22).

Based on the comparison in step S22, the controller 104 judges whether the operation information of the driver acquired in step S21 is included in the range of the pattern of normal operation information (step S23).

When the controller 104 judges that the operation information of the driver acquired in step S21 is included in the range of the pattern of normal operation information (step S23: Yes), the controller 104 judges that the degree of fatigue of the driver is low (step S26). In this case, the controller 104 transmits the judgment result to the server 200 (step S27).

On the other hand, when the controller 104 judges that the operation information of the driver acquired in step S21 is not included in the range of the pattern of normal operation information (step S23: No), the controller 104 judges that the degree of fatigue of the driver is high (step S24).

In this case, the controller 104 provides notification from the notification interface 106 that the degree of fatigue is high (step S25). The controller 104 may output a notification, from the notification interface 106, encouraging the driver to rest.

The controller 104 then transmits the judgment result to the server 200 (step S27).

In the flow illustrated in FIG. 6, the controller 104 has been described as providing notification from the notification interface 106 only when the operation information of the driver is not included in the range of the pattern of normal operation information (step S24: No). The controller 104 may, however, provide notification of the judgment result when the operation information of the driver is included in the range of the pattern of normal operation information (step S23: Yes).

In this way, the operation information of the driver is acquired by sensors included in the information acquisition interface 101, and the degree of fatigue of the driver is judged based on the operation information in the information processing system 1 according to the present embodiment. For example, the vehicle 100 acquires information related to a brake operation by the driver as the operation information and judges the degree of fatigue of the driver based on the brake operation, as described above. The degree of fatigue of the driver is judged by the information processing system 1 in this way.

The information processing system 1 provides notification of information related to the judged degree of fatigue from the notification interface 106. The driver can learn what his own degree of fatigue is by the notification from the notification interface 106. When the driver is notified of a high degree of fatigue by the notification interface 106, the driver can therefore take measures to ensure safety, such as resting, even if the driver is not conscious of being fatigued. The driver can thus take measures in advance. This makes it easier to prevent accidents and the like and improves safety.

The operation information of the driver is acquired by the sensors of the information acquisition interface 101 mounted in the vehicle 100. The information processing system 1 can therefore acquire the movement of the driver's body without the driver wearing sensors or the like on the body, for example. Consequently, the information processing system 1 can acquire operation information of the driver without placing a cumbersome burden for wearing on the driver. Operation information can also be acquired without the driver realizing that data is being acquired. Furthermore, the detection accuracy of data may decrease for reasons such as misalignment of sensors when, for example, the driver wears sensors or the like on the body. This problem does not occur, however, in the information processing system 1.

Various embodiments have been described for a complete and clear disclosure. The appended claims, however, are not limited to the above embodiments and are to be construed as encompassing all of the possible modifications and alternate configurations that a person of ordinary skill in the art could make within the scope of the fundamental features illustrated in the present disclosure. The subject matter of the various embodiments may also be freely combined.

For example, the operation information acquired by the information acquisition interface 101 during the test drive has been described in the above embodiment as being stored in the storage 103 as a pattern of normal operation information. However, the process to store the pattern of normal operation information is not limited to the example in the above embodiment. The process to store the normal pattern may be executed using a method such as deep learning.

When a brake operation is performed during the test drive, for example, the controller 104 may cause a display of the vehicle 100 to display a prompt for the driver to input whether the brake operation exhibits a pattern of normal operation information. The driver uses the input interface 105 to input whether the brake operation that the driver performed exhibits a pattern of normal operation information. When the driver thinks that the brake operation exhibits a typical operation, the driver can input that the brake operation exhibits a pattern of normal operation information. When the driver thinks that he braked hard, for example, he can input that the brake operation does not exhibit a pattern of normal operation information. The controller 104 may store (accumulate) the pattern of operation information in the storage 103 based on the driver input.

For example, the controller 104 in the above embodiment has been described as judging the degree of fatigue based on the operation information acquired by the information acquisition interface 101 and the pattern of operation information of the driver stored in the storage 103. The controller 104 may, however, judge the degree of fatigue resulting from the driver driving the vehicle 100, for example. Specifically, the controller 104 may store operation information acquired by the information acquisition interface 101 at a predetermined time after the driver starts to drive the vehicle 100 (such as five minutes after the start of driving) in the storage 103. The controller 104 acquires operation information with the information acquisition interface 101 while the driver is driving the vehicle 100 and compares the acquired operation information with the operation information acquired at the predetermined time after the start of driving. The controller 104 may judge the degree of fatigue of the driver based on the result of the comparison. Based on the comparison, the controller 104 may, for example, judge that the degree of fatigue is higher as the speed with which the brake pedal is pressed is faster. When the degree of fatigue exceeds a predetermined threshold, for example, the controller 104 may provide notification from the notification interface 106 that the degree of fatigue is high. The controller 104 can judge the degree of fatigue of the driver in this way based on the change in operation information from when the driver starts driving. In other words, the controller 104 can judge the degree of fatigue of the driver due to driving.

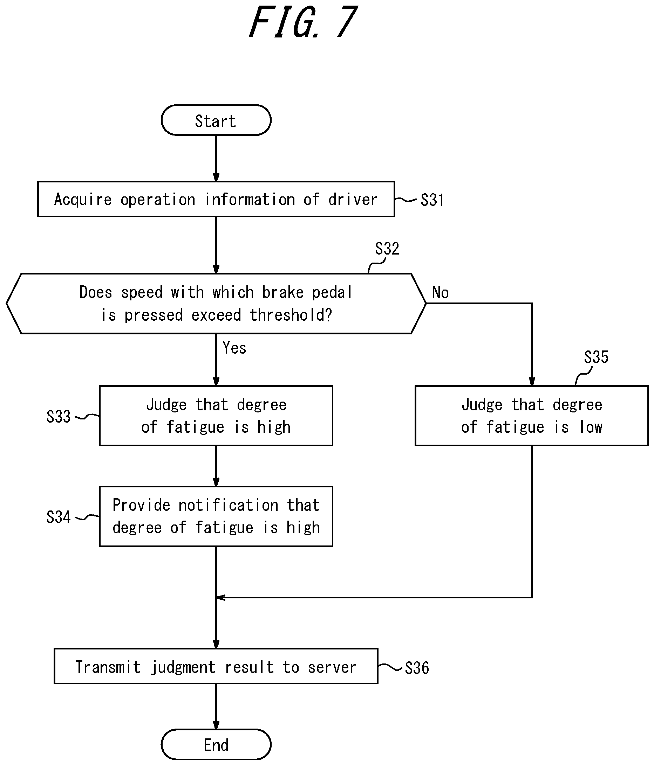

The controller 104 may, for example, judge the degree of fatigue of the driver by making a comparison with a threshold instead of or in addition to the comparison with the pattern. FIG. 7 is a flowchart illustrating a modification to the process executed by the controller 104 of the vehicle 100. FIG. 7 illustrates an example of judging the degree of fatigue of the driver by comparison with a threshold.

The controller 104 acquires operation information of the driver, detected by the sensors of the information acquisition interface 101, from the sensors while the driver is driving the vehicle 100 (step S31). For example, the controller 104 acquires information related to the speed with which the brake pedal is pressed, detected by the control speed sensor 125, as the operation information.

Based on the operation information of the driver acquired in step S21, the controller 104 judges whether the speed with which the driver presses the brake pedal exceeds a predetermined threshold (step S32). The predetermined threshold may be stored in the storage 103 in advance, for example. The predetermined threshold may be determined by the controller 104 based on operation information acquired when the driver takes a test drive, for example. The predetermined threshold may be a threshold allowing judgment of whether the driver is fatigued to the point that he may cause a traffic accident.

When the controller 104 judges that the speed with which the brake pedal is pressed does not exceed the threshold (step S32: No), the controller 104 judges that the degree of fatigue of the driver is low (step S35). In this case, the controller 104 transmits the judgment result to the server 200 (step S36).

Conversely, when the controller 104 judges that the speed with which the brake pedal is pressed exceeds the threshold (step S32: Yes), the controller 104 judges that the degree of fatigue of the driver is high (step S33).

In this case, the controller 104 provides notification from the notification interface 106 that the degree of fatigue is high (step S34). The controller 104 may output a notification, from the notification interface 106, encouraging the driver to rest.

The controller 104 then transmits the judgment result to the server 200 (step S36).

The controller 104 may also provide notification of the judgment result when the degree of fatigue of the driver is judged to be low (step S35). In this way, the controller 104 can judge the degree of fatigue based on a comparison other than a comparison with a pattern.

For example, the controller 104 has been described as judging the degree of fatigue of the driver based on the speed with which the brake pedal is pressed in the above embodiment. The controller 104 can, however, judge the degree of fatigue of the driver using a different standard. For example, the controller 104 can judge the degree of fatigue of the driver based on the frequency of hard braking. Specifically, the controller 104 can judge the degree of fatigue of the driver based on the ratio of the number of times a brake operation is judged to be hard braking to the number of times the driver presses the brake pedal, for example. The brake operation judged to be hard braking is, for example, a brake operation in which the brake pedal is pressed with a speed equal to or greater than a predetermined threshold. The controller 104 can judge that the degree of fatigue of the driver is higher as the aforementioned ratio is higher. When the degree of fatigue exceeds a predetermined threshold, for example, the controller 104 may provide notification from the notification interface 106 that the degree of fatigue is high.

For example, the operation information is information related to the speed with which the brake pedal is pressed, and the control speed sensor 125 acquires this operation information in the above embodiment. The operation information is not limited to this example, however, and may be any operation information acquirable by the sensors of the information acquisition interface 101. For example, the operation information may include the force with which the pedal is pressed, the force with which the handlebar is gripped, the speed or acceleration of the vehicle 100, the turning of the handlebar, the timing at which the clutch lever and the shift pedal are operated, and the like. The operation information may, for example, include the posture, gaze, or the like of the driver. The controller 104 may calculate an index, such as an average or a deviation, for a numerical value included in the operation information and judge the degree of fatigue using the calculated index.

The controller 104 may judge the degree of fatigue of the driver using a plurality of types of operation information. For example, the controller 104 may use two or more types of the following operation information to judge the degree of fatigue of the driver comprehensively: the speed with which the brake pedal is pressed, the force with which the pedal is pressed, the force with which the handlebar is gripped, the speed or acceleration of the vehicle 100, the turning of the handlebar, and the timing at which the clutch lever and the shift pedal are operated. The controller 104 may use a predetermined algorithm to judge the degree of fatigue of the driver by weighting the two or more types of operation information.

The processes that the controller 104 of the vehicle 100 has been described as executing in the above embodiment do not necessarily need to be executed by the controller 104 of the vehicle 100. For example, the storage process and the judgment process may be executed by the controller 202 of the server 200. In this case, movement of the driver's body acquired by the information acquisition interface 101 is transmitted from the vehicle 100 to the server 200. The controller 202 can execute the above-described storage process and judgment process on the server 200. In this case, the normal pattern may be stored by the storage 201 of the server 200.

### REFERENCE SIGNS LIST

- 1: Information processing system
- 100: Vehicle
- 101: Information acquisition interface
- 103, 201: Storage
- 104, 202: Controller
- 104a, 202a: Processor
- 105: Input interface
- 106: Notification interface
- 107, 203: Communication interface
- 110: Body
- 111: Front wheel
- 112: Rear wheel
- 113: Handlebar
- 114: Seat
- 115: Pedal
- 121: Pressure sensor
- 122: Imaging unit
- 123: Motion sensor
- 124: Vehicle speed sensor
- 125: Control speed sensor
- 200: Server

## Claims

1. A vehicle comprising:
an information acquisition interface configured to acquire operation information resulting from a control action performed by a driver; and
a controller configured to judge a degree of fatigue of the driver based on the operation information.

2. The vehicle of claim 1, wherein the operation information includes information related to at least one of a steering operation, an accelerator operation, a brake operation, and a gear operation of the vehicle.

3. The vehicle of claim 2, wherein the information related to the brake operation includes information related to a speed at which the brake is operated.

4. The vehicle of claim 1, further comprising:
a storage configured to store a pattern of the operation information resulting from the control action performed by the driver;
wherein the controller is configured to judge the degree of fatigue of the driver based on whether the operation information acquired by the information acquisition interface is included in a range of the pattern, stored in the storage, of the operation information resulting from the control action performed by the driver.

5. The vehicle of claim 1, further comprising:
a notification interface configured to provide notification of information;
wherein the controller is configured to provide notification of information related to the degree of fatigue of the driver from the notification interface.

6. A judgment method comprising:
acquiring, by a controller using an information acquisition interface, operation information resulting from a control action performed by a driver; and
judging, by the controller, a degree of fatigue of the driver based on the operation information.

7. A judgment program for causing a computer to:
acquire operation information resulting from a control action performed by a driver; and
judge a degree of fatigue of the driver based on the acquired operation information.
